(19) Europäisches Patentamt
European Patent Office
Office européen des brevets

(11) Veröffentlichungsnummer : **0 416 359 B1**

# (12) EUROPÄISCHE PATENTSCHRIFT

(45) Veröffentlichungstag der Patentschrift : **07.07.93 Patentblatt 93/27**

(51) Int. Cl.$^5$ : **C07C 271/44,** C07C 271/48, A01N 47/22

(21) Anmeldenummer : **90115949.1**

(22) Anmeldetag : **21.08.90**

## (54) Carbocyclische Anilid-Carbamate.

Die Akte enthält technische Angaben, die nach dem Eingang der Anmeldung eingereicht wurden und die nicht in dieser Patentschrift enthalten sind.

(30) Priorität : **02.09.89 DE 3929232**
**21.04.90 DE 4012791**

(43) Veröffentlichungstag der Anmeldung : **13.03.91 Patentblatt 91/11**

(45) Bekanntmachung des Hinweises auf die Patenterteilung : **07.07.93 Patentblatt 93/27**

(84) Benannte Vertragsstaaten : **BE CH DE FR GB IT LI NL**

(56) Entgegenhaltungen :
**EP-A- 0 116 409**
**EP-A- 0 293 718**
**US-A- 4 051 254**

(73) Patentinhaber : **BAYER AG**
**W-5090 Leverkusen 1 Bayerwerk (DE)**

(72) Erfinder : **Krüger, Bernd-Wieland, Dr.**
**Unterboschbach 19**
**W-5060 Bergisch Gladbach 2 (DE)**
Erfinder : **Sasse, Klaus, Dr.**
**Pützweg 13**
**W-5060 Bergisch Gladbach 2 (DE)**
Erfinder : **Brandes, Wilhelm, Dr.**
**Eichendorffstrasse 3**
**W-5653 Leichlingen 1 (DE)**

EP 0 416 359 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Cycloalkyl- bzw. Cycloalkenylcarbonsäureanilidcarbamate, ein Verfahren zu ihrer Herstellung und ihre Verwendung zur Bekämpfung von Schädlingen, insbesondere von Pilzen.

Es ist bekannt, daß bestimmte substituierte 3-Aminophenyl-carbamate herbizide Eigenschaften besitzen (vgl. US-A 3 832 384).

Weiterhin sind viele Phenylcarbamate bekannt, die eine fungizide Wirkung besitzen (vgl. EP-A 116 409; EP-A 117 024; EP-A 125 901 und EP-A 293 718).

Es werden aber keine Verbindungen dieses Typs offenbart, in denen am Stickstoffatom der Carbamat-Gruppe ein Alkyl- oder Alkylcarbonyloxyalkyl-Rest enthalten ist und gleichzeitig in para-Stellung zur Carbamat-Gruppe ein substituierter Cycloalkylrest vorhanden ist, der über -CO-NH- mit dem Phenylring verbunden ist.

Weiterhin sind viele Carbonsäureanilide mit fungizider Wirkung, insbesondere mit Wirkung gegen Benzimidazoltolerante Pflanzenpathogene, bekannt (vgl. EP-A 100 615).

Es wurden neue Cycloalkyl- bzw. Cycloalkenyl-carbonsäureanilidcarbamate der Formel

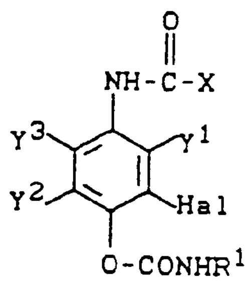

(I)

gefunden,
in welcher

X für ein- bis sechsfach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht oder für Cycloalkenyl mit 5 - 7 Ringgliedern steht, wobei der Cycloalkenylrest ein- bis sechsfach, gleich oder verschieden durch geradkettiges oder verzweigtes Alkyl mit 1 - 4 Kohlenstoffatomen substituiert sein kann,

Hal für Fluor, Chlor oder Brom steht,

$Y^1$, $Y^2$ und $Y^3$ gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 - 4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen, und

$R^1$ für $C_1$-$C_8$-Alkyl oder $C_1$-$C_8$-Alkylcarbonyloxy-$C_1$-$C_6$-alkyl steht.

Die Cycloalkyl- bzw, Cycloalkenyl-carbonsäureanilidcarbamate der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen Cycloalkyl- bzw. Cycloalkenylcarbonsäureanilidcarbamate der Formel

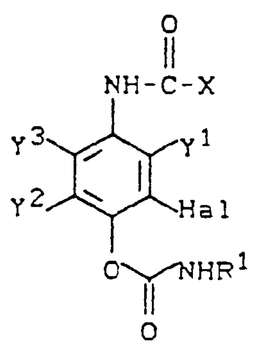

(I)

in welcher

X, Hal, $Y^1$, $Y^2$, $Y^3$ und $R^1$ die oben angegebenen Bedeutungen haben,

erhält, wenn man Aminophenole der Formel

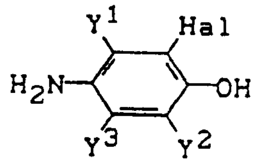

(II)

in welcher

Hal, $Y^1$, $Y^2$ und $Y^3$ die oben angegebenen Bedeutungen haben, in einem ersten Reaktionsschritt mit Carbonsäure-Derivaten der Formel

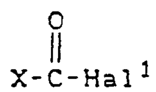

(III)

in welcher

X die oben angegebene Bedeutung hat und

$Hal^1$ für Halogen, vorzugsweise Chlor, oder eine bei Acylierungsreaktionen gebräuchliche Abgangsgruppe, vorzugsweise einen aktivierenden Esterrest, steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt die so erhaltenen Zwischenprodukte der Formel

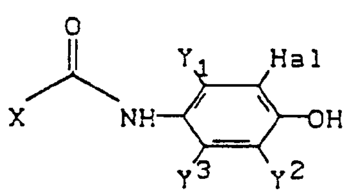

(IV)

in welcher

X, $Y^1$, $Y^2$, $Y^3$ und Hal die obengenannte Bedeutung haben,

mit Isocyanaten der Formel

$R^1$-NCO (V)

in welcher

$R^1$ die obengenannte Bedeutung hat, gegebenenfalls in Gegenwart einer Base und gegebenenfalls in Gegenwart von Verdünnungs- oder Lösungsmitteln umsetzt.

Schließlich wurde gefunden, daß die Cycloalkyl- bzw. Cycloalkenyl-carbonsäureanilidcarbamate der Formel (I) u. a. eine hohe fungizide Wirksamkeit besitzen. Die neuen Verbindungen können auch mit anderen bekannten, hochwirksamen Verbindungen in synergistischen Mischungen verwendet werden.

Die erfindungsgemäßen Cycloalkyl- bzw. Cycloalkenyl-carbonsäureanilid-carbamate sind durch die Formel (I) allgemein definiert. Bevorzugt sind Verbindungen der Formel (I), worin

X für ein- oder zweifach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl, für gegebenenfalls ein- oder zweifach, gleich oder verschieden durch geradkettiges oder verzweigtes Alkyl mit 1 - 4 Kohlenstoffatomen substituiertes Cyclopentenyl, Cyclohexenyl, Cycloheptenyl steht,

$Y^1$, $Y^2$ und $Y^3$ gleich oder verschieden sind und für Wasserstoff, Fluor, Chlor, Brom, Methyl oder Trifluormethyl stehen und

Hal für Fluor, Chlor oder Brom steht, und

$R^1$ für $C_1$-$C_6$-Alkyl oder $C_1$-$C_6$-Alkylcarbonyloxy-$C_1$-$C_6$-alkyl steht.

Besonders bevorzugt sind Verbindungen der Formel (I), worin

X für in 1- oder 1,3-Stellung durch Methyl oder Ethyl substituiertes Cyclopentyl, Cyclohexyl, Cycloheptyl, Cyclopentenyl, Cyclohexenyl, Cycloheptenyl steht, welche gegebenenfalls durch einen weiteren Alkylrest mit 1 - 3 Kohlenstoffatomen zusätzlich substituiert sind,

Hal für Fluor, Chlor oder Brom steht,

$Y^1$, $Y^2$ und $Y^3$ gleich oder verschieden sind und für Wasserstoff, Fluor, Chlor, Brom oder Trifluormethyl stehen und

$R^1$ für $C_1$-$C_4$-Alkyl oder $C_1$-$C_5$-Alkylcarbonyloxy-$C_1$-$C_5$-alkyl steht.

Verwendet man beispielsweise 2,6-Dichlor-4-amino-phenol, 1-Methyl-1-chlorcarbonylcyclohexan und 3-Acetyl-oxypropylisocyanat als Ausgangsstoffe, so kann der Reaktionsverlauf durch das folgende Formelschema wiedergegeben werden:

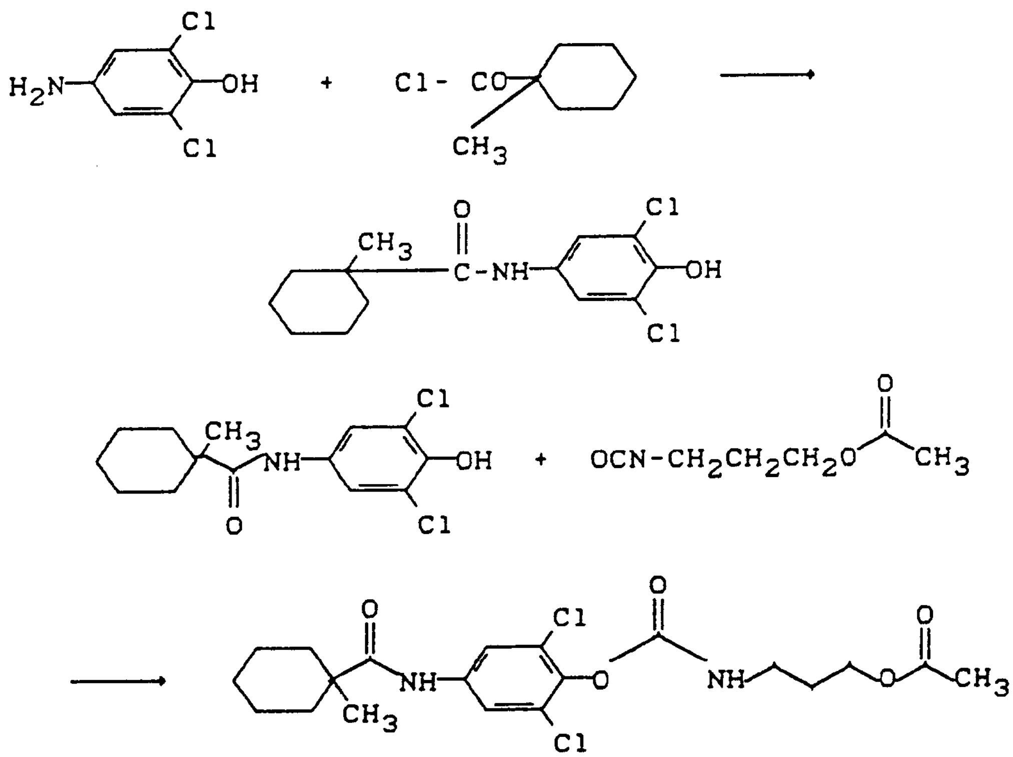

Die zur Durchführung des erfindungsgemäßen Verfahrens als Ausgangsstoffe benötigten Aminophenole sind durch die Formel (II) allgemein definiert. In dieser Formel (II) haben die Reste Hal und $Y^1$ - $Y^3$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar (vergl. "Methoden der organischen Chemie", Houben-Weyl, Band VI/1c, Phenole, Teil 1, Georg Thieme Verlag, Stuttgart, 1976, und "Reaktionen der organischen Synthese", Cesare Ferri, S. 81, 89, 91, 97, 118, 120, 122, 124, 126, 128, Georg Thieme Verlag, Stuttgart, 1978).

Die 4-Amino-2-chlor- bzw. -2-brom-6-trifluormethylpbenole sind bekannt aus Jp. Kokai Tokkyo Koho Jp 61/126055 und z. B. 4-Amino-2,3,5,6-tetrafluorphenol aus Zh. Org. Khim. 10(9), 1923-1927 (1974). Die Verbindungen der Formel

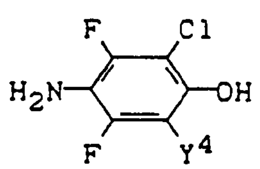

(II A)

in welcher

$Y^4$ für Fluor oder Chlor steht, sind Gegenstand von EP-A-293 718 und können z. B. hergestellt werden aus entsprechenden Hydroxybenzoesäuren der Formel

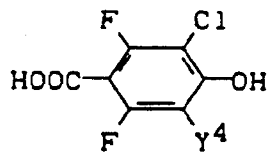

(VA)

durch Decarboxylierung mit anschließender Nitrierung der entstehenden Phenole der Formel

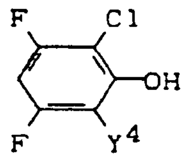

(VI A)

zu den Nitroverbindungen der Formel

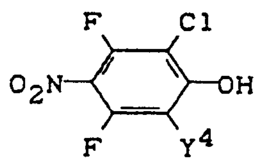

(VII A)

die dann hydriert werden, z. B. mit Wasserstoff und Raney-Nickel, zu den entsprechenden Aminen der Formel (II A).

Auch die Verbindungen der Formel (VII A) sind Gegenstand der EP-A-293 718.

Die zur Durchführung des erfindungsgemäßen Verfahrens außerdem benötigten Cycloalkancarbonsäure- bzw. Cycloalkencarbonsäure-Derivate sind durch die Formel (III), in der X für Cycloalkyl oder Cycloalkenyl steht, allgemein definiert. In dieser Formel (III) haben die Reste X und $Hal^1$ die bereits im Zusammenhang mit der Beschreibung der erfindungsgemäßen Verbindungen der Formel (I) gegebenen Bedeutungen. Die Verbindungen sind bekannt und nach Analogieverfahren herstellbar (vergl. Diversi et. al., Synthesis 1971, 258; US 3 674 831; "Reaktionen der organischen Synthese" Cesare Ferri, S. 460, 461, 1978, Georg Thieme Verlag, Stuttgart; Houben-Weyl, Methoden der organischen Chemie, Bd. 5 Tl.1, S.211, 320, 343, 428 ff, G. Thieme-Verlag, Stuttgart, 1985).

Die zur Durchführung des erfindungsgemäßen Verfahrens außerdem benötigten Isocyanate, in welcher $R^1$ die oben angegebene Bedeutung hat, sind bekannt oder nach Analogieverfahren herstellbar (vgl. Methoden der organischen Chemie, Houben-Weyl, Bd. E4, Kohlensäure-Derivate, Georg Thieme Verlag, Stuttgart, S. 738 ff (1983)).

Die bei dem erfindungsgemäßen Verfahren als Zwischenprodukte verwendeten Acylaminophenole sind durch die Formel (IV) definiert.

Die Verbindungen der Formel (IV) sind teilweise neu, und werden in einer parallelen Anmeldung beansprucht.

Man erhält die Acylamino-Derivate der Formel (IV), indem man Aminophenole der Formel

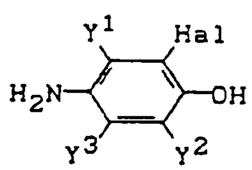

(II)

in welcher

$Y^1$, $Y^2$, $Y^3$ und Hal die obengenannte Bedeutung haben, mit Carbonsäure-Derivaten der Formel

$$X-\overset{\overset{\displaystyle O}{\|}}{C}-Hal^1 \qquad (III)$$

in welcher
X und $Hal^1$ die obengenannte Bedeutung haben,
gegebenenfalls in Gegenwart eines Lösungsmittels und gegebenenfalls in Gegenwart eines Säureakzeptors umsetzt.

Das erfindungsgemäße Verfahren wird gegebenenfalls in Gegenwart von Säureakzeptoren bzw. Basen durchgeführt. Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. - ethylat, ferner aliphatische, aromatische und heterocyclische Amine, beispielsweise Triethylamin, Trimethylamin, Dimethylanilin, 1,8-Diazabicyclo-(5,4,0)undec-7-en, Dimethylbenzylamin und Pyridin.

Zur Durchführung des erfindungsgemäßen Verfahrens setzt man im ersten Reaktionsschritt vorzugsweise auf ein Mol Aminophenol der allgemeinen Formel (II) 1 - 2 Mol, insbesondere 1 - 1,4 Mol, der Verbindungen der allgemeinen Formel (III) ein.

Für den zweiten Reaktionsschritt des erfindungsgemäßen Verfahrens setzt man vorzugsweise auf 1 Mol Acylaminophenol der Formel (IV) 1-2 Mol, insbesondere 1-1,4 Mol, der Verbindungen der allgemeinen Formel (V) ein.

Als Verdünnungsmittel zur Durchführung des erfindungsgemäßen Verfahrens kommen praktisch alle inerten organischen Verdünnungsmittel in Frage. Hierzu gehören insbesondere aliphatische und aromatische, gegebenenfalls halogenierte Kohlenwasserstoffe wie Pentan, Hexan, Heptan, Cyclohexan, Petrolether, Benzin, Ligroin, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methylisopropyl- und Methylisobutylketon, Ester wie Essigsäuremethylester und - ethylester, Nitrile wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Das erfindungsgemäße Verfahren wird im allgemeinen bei Temperaturen zwischen -50° C und 120° C durchgeführt. Bevorzugt wird der Bereich zwischen 0° C und 110° C. Die Umsetzungen werden im allgemeinen bei Normaldruck durchgeführt.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillieren", d. h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z. B. durch Kristallisation. Zur Charakterisierung der Verbindungen dienen Brechungsindex, Schmelzpunkt, $R_f$-Wert oder Siedepunkt.

Die erfindungsgemäßen Wirkstoffe sind für den Gebrauch zur Bekämpfung von Schädlingen, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv, oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;

Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Bebandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Botrytispilzen an Bohnen sowie zur Bekämpfung von Reiskrankheiten, wie beispielsweise gegen den Erreger der Reisfleckenkrankheit (Pyricularia oryzae) einsetzen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel 1

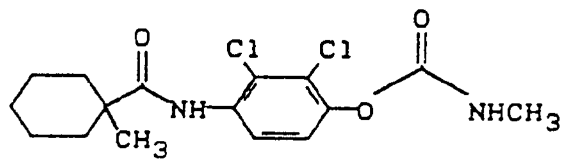

4 g (13,0 mMol) 2,3-Dichlor-4-(1-methyl-cyclohexylcarbonylamino)-phenol werden in 20 ml Toluol gelöst und zwischen 10 und 20° C mit 0,9 g (19,5 mMol) Methylisocyanat versetzt. Anschließend gibt man 30 mg DBU-(1,8-Diazabicyclo-(5,4,0)-undec-7-en)zur Reaktionsmischung. Man rührt zwei Stunden bei 20° C und setzt 1,0 g (21,7 mMol) Methylisocyanat zu. Nach beendeter Reaktion (DC-Kontrolle) wird abgekühlt und langsam mit n-Hexan versetzt. Der ausgefallene Feststoff wird abgesaugt und mit Hexan gewaschen. Ausbeute: 3,5 g (81 % d.Th.). Fp. 140° C.

Analog Beispiel 1 werden die folgenden Verbindungen der Formel (I) hergestellt:

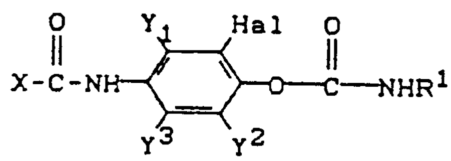

(I)

| Beispiel Nr. | X | $Y^1$ | $Y^3$ | $Y^2$ | Hal | $R^1$ | phys. Daten Fp. |
|---|---|---|---|---|---|---|---|
| 2 | 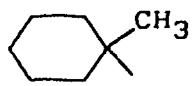 | Cl | H | H | Cl | $CH_2C(CH_3)_3$ | 121° C |
| 3 | 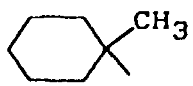 | Cl | H | H | Cl | $C(CH_3)_3$ | 118° C |
| 4 | 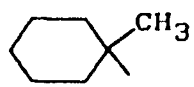 | H | H | Cl | Cl | $C(CH_3)_3$ | 131° C |
| 5 | 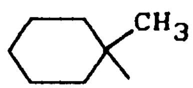 | H | H | Cl | Cl | $CH_2C(CH_3)_3$ | 140° C |
| 6 | 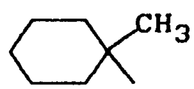 | H | H | Cl | Cl | $CH_2C(CH_3)_2CH_2OCOCH_3$ | 35° C |
| 7 | 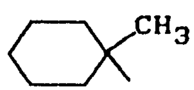 | H | H | Cl | Cl | $CH_2CH(CH_3)OCOCH_3$ | 93° C |

| Beispiel Nr. | X | $Y^1$ | $Y^3$ | $Y^2$ | Hal | $R^1$ | phys. Daten Fp. |
|---|---|---|---|---|---|---|---|
| 8 | 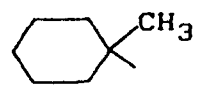 | H | H | Cl | Cl | $CH_2CH(CH_3)OCOC_4H_9$-t | 106° C |
| 9 | 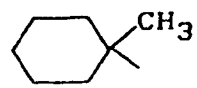 | H | H | Cl | Cl | $CH_2C(CH_3)_2CH_2OCOC_4H_9$-t | 38° C |
| 10 | 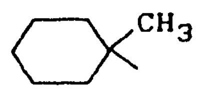 | H | H | Cl | Cl | $CH_2CH_2OCOC_4H_9$-t | 132° C |
| 11 | 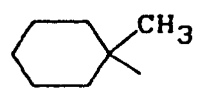 | H | H | Cl | Cl | $CH_2CH_2OCOCH_3$ | 119° C |
| 12 | 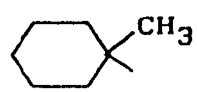 | Cl | H | H | Cl | $CH_2CH(CH_3)OCOC_4H_9$-t | 103° C |
| 13 | 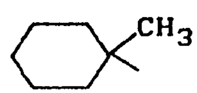 | Cl | H | H | Cl | $CH_2C(CH_3)_2CH_2OCOC_4H_9$-t | 92° C |

| Beispiel Nr. | X | $Y^1$ | $Y^3$ | $Y^2$ | Hal | $R^1$ | phys. Daten Fp. |
|---|---|---|---|---|---|---|---|
| 14 | 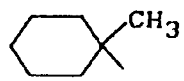 | Cl | H | H | Cl | $CH_2CH_2OCOC_4H_9$-t | 125° C |
| 15 | 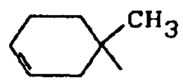 | Cl | H | H | Cl | $CH_3$ | |
| 16 | 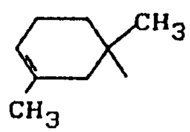 | Cl | H | H | Cl | $CH_3$ | |
| 17 | 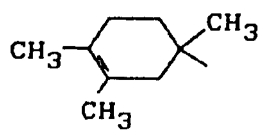 | Cl | H | H | Cl | $CH_3$ | |
| 18 | 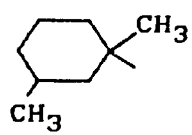 | Cl | H | H | Cl | $CH_3$ | |
| 19 | 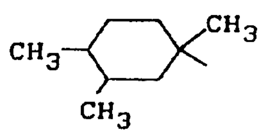 | Cl | H | H | Cl | $CH_3$ | |

| Beispiel Nr. | X | $Y^1$ | $Y^3$ | $Y^2$ | Hal | $R^1$ | phys. Daten Fp. |
|---|---|---|---|---|---|---|---|
| 20 | 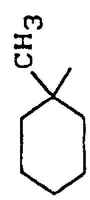 | F | F | F | Cl | $CH_3$ | |

## Herstellungsbeispiele

Herstellung der Ausgangsverbindungen

Beispiel A1:

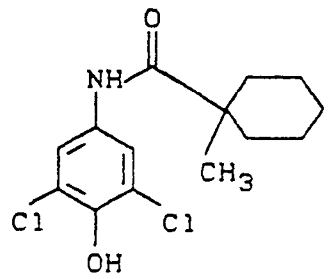

18,5 g (0,085 Mol) 4-Amino-2,6-dichlorphenol werden in 150 ml Tetrahydrofuran gelöst und zunächst mit 8,6 g (0,085 Mol) Triethylamin, dann bei 0° C Innentemperatur mit 15 g (0,094 Mol) 1-Methyl-cyclohexancarbonsäurechlorid versetzt. Man rührt über Nacht bei 20° C und fügt dann zur Vervollständigung der Reaktion erneut 5 g Carbonsäurechlorid und 2,8 g Triethylamin zur Reaktionsmischung. Nach 2 Stunden wird auf Eis gegossen und der abgesaugte Feststoff aus Toluol umkristallisiert. Man erhält die obengenannte Verbindung mit dem Schmelzpunkt 140° C; Ausbeute: 22,3 g (= 87 % der Theorie).

Beispiel A2

3,5-Dichlor-2,6-difluor-4-hydroxybenzoesäure

In einer Rührapparatur werden 300 g Kaliumhydroxid, 600 ml Wasser, 15 g Tetrabutylammoniumchlorid und 135 g 3,5-Dichlor-2,4,6-trifluorbenzotrifluorid vorgelegt und dann für 5 Stunden am Rückfluß erhitzt. Nach Ende der Reaktion wird abgekühlt und durch Zutropfen von Salzsäure sauer gestellt. Das Festprodukt wird abgesaugt und im Vakuum getrocknet. Ausbeute: 93 g mit einem Schmelzpunkt 102 - 105° C.

Beispiel A3

3-Chlor-2,5,6-trifluor-4-hydroxy-benzoesäure

Analog Beispiel A1 werden aus 400 g NaOH, 1200 ml Wasser, 15 g Tetraethylammoniumchlorid und 276 g 3-Chlortetrafluorbenzotrifluorid bei 6-stündigem Erhitzen unter Rückfluß 238 g Produkt erhalten mit einem Schmelzpunkt von 87 - 90° C.

Beispiel A4

2,6-Dichlor-3,5-difluorphenol

50 g 3,5-Dichlor-2,6-difluor-4-hydroxy-benzoesäure und 10 ml Dimethylformamid werden vermischt und erhitzt. Bei 105 - 130° C entwickelt sich Kohlendioxid und man läßt bei dieser Temperatur ausreagieren. Anschließend rührt man 200 ml Toluol und danach 80 ml Wasser ein, trennt die Phasen, trocknet die organische Phase und destilliert anschließend. Man erhält 34 g des Produktes mit einem Siedepunkt von 87 - 88° C und einem Brechungsindex von $n_D^{20}$: 1,5310.

Beispiel A5

Analog Beispiel A3 erhält man 2-Chlor-3,5,6-trifluorphenol, mit einem Siedepunkt 68 - 70° C / 20 mbar.

Beispiel A6

2,6-Dichlor-3,5-difluor-4-nitro-phenol

In 70 ml Essigsäure werden 20 g 2,6-Dichlor-3,5-difluorphenol vorgelegt und 8 g 98 %ige Salpetersäure zugetropft. Anschließend wird für 2 Stunden bei Raumtemperatur nachgerührt, in 150 ml Dichlormethan aufgenommen und zweimal mit Wasser gewaschen. Nach Abdestillieren des Dichlormethans verbleiben 18 g Produkt. Nach GC-Analyse 94 %ig.

Beispiel A7

2-Chlor-3,5,6-trifluor-4-nitrophenol

Analog Beispiel A5 werden durch Nitrierung aus 28 g 2-Chlor-3,5,6-trifluorphenol 25 g 2-Chlor-3,5,6-trifluor-4-nitrophenol erhalten mit einer Reinheit von 93 % und einem Schmelzpunkt von 107 - 109° C.

Beispiel A8

2,6-Dichlor-3,5-difluor-4-amino-phenol

18 g 2,6-Dichlor-3,5-difluor-4-nitrophenol werden in 100 ml Methanol in Gegenwart von 1,5 g Raney-Nickel bei 25 - 45° C mit 30 - 50 bar Wasserstoff bis zum ende der Wasserstoffaufnahme hydriert. Nach Filtration wird die Lösung unter vermindertem Druck vom Lösungsmittel befreit. Es verbleiben 13 g Aminophenol (GC-Reinheit 98,4 %); Fp. 151° C.

Beispiel A9

2-Chlor-3,5,6-trifluor-4-amino-phenol

Analog Beispiel A7 werden aus 25 g 2-Chlor-3,5,6-trifluor-4-nitro-phenol in 120 ml Methanol und 2 g Raney-Nickel durch Hydrierung 20 g Aminophenol (GC-Reinheit 97 %) erhalten.

Analog Beispiel A1 werden die folgenden Verbindungen der Formel (IV) erhalten.

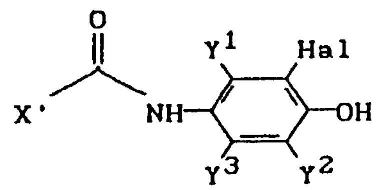

(IV)

| Beispiel-Nr. | X' | $Y^1$ | $Y^3$ | $Y^2$ | Hal | phys. Daten |
|---|---|---|---|---|---|---|
| A11 | 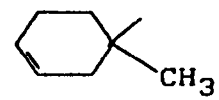 | Cl | H | H | Cl | |
| A12 | 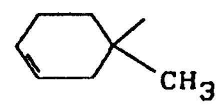 | Cl | H | Cl | H | |
| A13 | 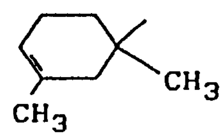 | Cl | H | H | Cl | |
| A14 | 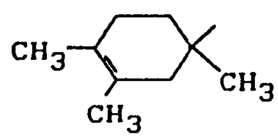 | Cl | H | H | Cl | |

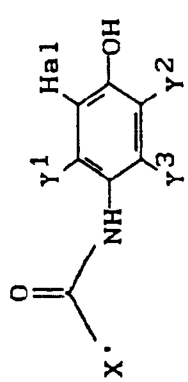

(IV)

| Beispiel-Nr. | X' | $Y^1$ | $Y^3$ | $Y^2$ | Hal | phys. Daten |
|---|---|---|---|---|---|---|
| A15 | 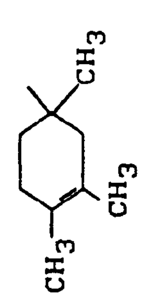 | Cl | H | Cl | H | |
| A16 | 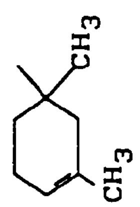 | Cl | H | Cl | H | |

Beispiel

Botrytis-Test (Bohne)/protektiv

Lösungsmittel: 4,7 Gewichtsteile Aceton

Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer

bei 20° C aufgestellt. 3 Tage nach der Inokulation wird die Größe der Befallsflecken auf den Blättern ausgewertet.

Eine ausgezeichnete Wirksamkeit zeigen in diesem Test z.B. die Verbindungen gemäß folgender Herstellungsbeispiele: 1, 7, 10, 11, 13 und 14.

## Patentansprüche

1. Cycloalkyl- bzw. Cycloalkenyl-carbonsäureanilidcarbamate der Formel

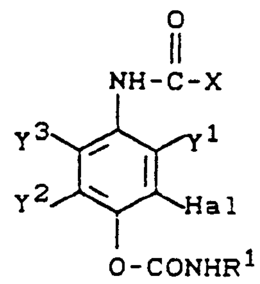

(I)

in welcher

| | |
|---|---|
| X | für ein- bis sechsfach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl steht oder für Cycloalkenyl mit 5 - 7 Ringgliedern steht, wobei der Cycloalkenylrest ein- bis sechsfach, gleich oder verschieden durch geradkettiges oder verzweigtes Alkyl mit 1 - 4 Kohlenstoffatomen substituiert sein kann, |
| Hal | für Fluor, Chlor oder Brom steht, |
| $Y^1$, $Y^2$ und $Y^3$ | gleich oder verschieden sind und für Wasserstoff, Fluor, Chlor, Brom, geradkettiges oder verzweigtes Alkyl mit 1 - 4 Kohlenstoffatomen, geradkettiges oder verzweigtes Alkoxy oder Alkylthio mit je 1 - 4 Kohlenstoffatomen, für Halogenalkyl, Halogenalkoxy oder Halogenalkylthio mit je 1 - 4 Kohlenstoffatomen im geradkettigen oder verzweigten Alkylteil und mit 1 - 5 gleichen oder verschiedenen Halogenatomen stehen, und |
| $R^1$ | für $C_1$-$C_8$-Alkyl oder $C_1$-$C_8$-Alkylcarbonyloxy-$C_1$-$C_6$-alkyl steht. |

2. Verbindungen der Formel (I) gemäß Anspruch 1, worin

| | |
|---|---|
| X | für ein- oder zweifach, gleich oder verschieden durch geradkettiges oder verzweigtes Alkyl mit 1 - 4 Kohlenstoffatomen substituiertes Cyclopropyl, Cyclobutyl, Cyclopentyl, Cyclohexyl oder Cycloheptyl, für gegebenenfalls ein- oder zweifach, gleich oder verschieden durch geradkettiges oder verzweigtes Alkyl mit 1 - 4 Kohlenstoffatomen substituiertes Cyclopentenyl, Cyclohexenyl, Cycloheptenyl steht, |
| $Y^1$, $Y^2$ und $Y^3$ | gleich oder verschieden sind und für Wasserstoff, Fluor, Chlor, Brom, Methyl oder Trifluormethyl stehen und |
| Hal | für Fluor, Chlor oder Brom steht, und |
| $R^1$ | für $C_1$-$C_6$-Alkyl oder $C_1$-$C_6$-Alkylcarbonyloxy-$C_1$-$C_6$-alkyl steht. |

3. Verbindungen der Formel (I) gemäß Anspruch 1, worin

| | |
|---|---|
| X | für in 1- oder 1,3-Stellung durch Methyl oder Ethyl substituiertes Cyclopentyl, Cyclohexyl, Cycloheptyl, Cyclopentenyl, Cyclohexenyl, Cycloheptenyl steht, welche gegebenenfalls durch einen weiteren Alkylrest mit 1 - 3 Kohlenstoffatomen zusätzlich substituiert sind, |
| Hal | für Fluor, Chlor oder Brom steht, |
| $Y^1$, $Y^2$ und $Y^3$ | gleich oder verschieden sind und für Wasserstoff, Fluor, Chlor, Brom oder Trifluormethyl stehen und |
| $R^1$ | für $C_1$-$C_4$-Alkyl oder $C_1$-$C_5$-Alkylcarbonyloxy-$C_1$-$C_5$-alkyl steht. |

4. Verfahren zur Herstellung von Cycloalkyl- bzw, Cycloalkenylcarbonsäureanilidcarbamaten der Formel

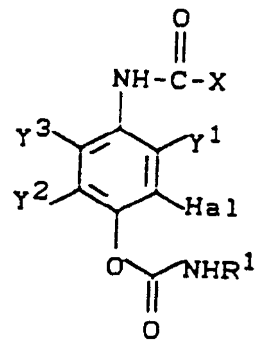

(I)

in welcher

X , Hal , $Y^1$, $Y^2$, $Y^3$ und $R^1$ die oben angegebenen Bedeutungen haben,

dadurch gekennzeichnet, daß man Aminophenole der Formel

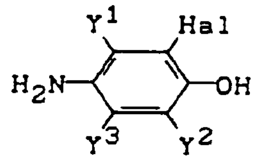

(II)

in welcher

Hal, $Y^1$, $Y^2$ und $Y^3$ die oben angegebenen Bedeutungen haben, in einem ersten Reaktionsschritt mit Carbonsäure-Derivaten der Formel

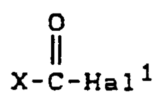

(III)

in welcher

X die oben angegebene Bedeutung hat und

$Hal^1$ für Halogen oder eine bei Acylierungsreaktionen gebräuchliche Abgangsgruppe steht,

gegebenenfalls in Gegenwart eines Säureakzeptors und gegebenenfalls in Gegenwart eines Lösungs- oder Verdünnungsmittels umsetzt, und dann in einem zweiten Reaktionsschritt die so erhaltenen Zwischenprodukte der Formel

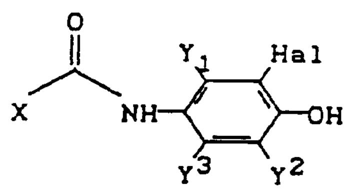

(IV)

in welcher

X, $Y^1$, $Y^2$, $Y^3$ und Mal die obengenannte Bedeutung haben,

mit Isocyanaten der Formel

$$R^1\text{-NCO} \qquad (V)$$

in welcher

$R^1$ die obengenannte Bedeutung hat, gegebenenfalls in Gegenwart einer Base und gegebenenfalls in Gegenwart von Verdünnungs- oder Lösungsmitteln umsetzt.

**5.** Schädlingsbekämpfungsmittel, gekennzeichnet durch einen Gehalt an mindestens einem Cycloalkyl- bzw. Cycloalkenyl-carbonsäureanilidcarbamat der Formel (I) gemäß Anspruch 1 .

6. Verwendung von Cycloalkyl- bzw. Cycloalkenyl-carbonsäureanilidcarbamaten der Formel (I) gemäß Anspruch 1 zur Bekämpfung von Schädlingen.

7. Verfahren zur Bekämpfung von Schädlingen, dadurch gekennzeichnet, daß man Cycloalkyl- bzw. Cycloalkenyl-carbonsäureanilidcarbamate der Formel (I) gemäß Anspruch 1 auf Schädlinge und/oder ihren Lebensraum einwirken läßt.

8. Verfahren zur Herstellung von Schädlingsbekämpfungsmitteln, dadurch gekennzeichnet, daß man Cycloalkyl- bzw. Cycloalkenyl-carbonsäureanilidcarbamate der Formel (I) gemäß Anspruch 1 mit Streckmitteln und/oder oberflächenaktiven Mitteln vermischt.

## Claims

1. Cycloalkyl- or cycloalkenyl-carboxanilide carbamates of the formula

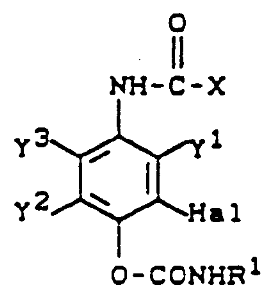

(I)

in which

| | |
|---|---|
| X | represents cyclopropyl, cyclobutyl, cyclopentyl, cyclohexyl or cycloheptyl, each of which is monosubstituted to hexasubstituted by identical or different substituents from the series comprising straight-chain or branched alkyl radicals having 1-4 carbon atoms, or represents cycloalkenyl having 5-7 ring members where the cycloalkenyl radical can be monosubstituted to hexasubstituted by identical or different substituents from the series comprising straight-chain or branched alkyl radicals having 1-4 carbon atoms, |
| Hal | represents fluorine, chlorine or bromine, |
| $Y^1$, $Y^2$ and $Y^3$ | are identical or different and represent hydrogen, fluorine, chlorine, bromine, straight-chain or branched alkyl having 1-4 carbon atoms, straight-chain or branched alkoxy or alkylthio each having 1-4 carbon atoms, or represent halogenoalkyl, halogenoalkoxy or halogenoalkylthio each having 1-4 carbon atoms in the straight-chain or branched alkyl moiety and 1-5 identical or different halogen atoms, and |
| $R^1$ | represents $C_1$-$C_8$-alkyl or $C_1$-$C_8$-alkylcarbonyloxy-$C_1$-$C_6$-alkyl. |

2. Compounds of the formula (I) according to Claim 1, where

| | |
|---|---|
| X | represents cyclopropyl, cyclobutyl, cyclopentyl, cyclohexyl or cycloheptyl, each of which is monosubstituted or disubstituted by identical or different substituents from the series comprising straight-chain or branched alkyl radicals having 1-4 carbon atoms, or represents cyclopentenyl, cyclohexenyl or cycloheptenyl, each of which is optionally monosubstituted or disubstituted by identical or different substituents from the series comprising straight-chain or branched alkyl radicals having 1-4 carbon atoms, |
| $Y^1$, $Y^2$ and $Y^3$ | are identical or different and represent hydrogen, fluorine, chlorine, bromine, methyl or trifluoromethyl, and |
| Hal | represents fluorine, chlorine or bromine, and |
| $R^1$ | represents $C_1$-$C_6$-alkyl or $C_1$-$C_6$-alkylcarbonyloxy-$C_1$-$C_6$-alkyl. |

3. Compounds of the formula (I), according to Claim 1, where

| | |
|---|---|
| X | represents cyclopentyl, cyclohexyl, cycloheptyl, cyclopentenyl, cyclohexenyl or cycloheptenyl, each of which is substituted in the 1- or 1,3-position by methyl or ethyl, and |

| | |
|---|---|
| | each of which is optionally additionally substituted by a further alkyl radical having 1-3 carbon atoms, |
| Hal | represents fluorine, chlorine or bromine, |
| $Y^1$, $Y^2$ and $Y^3$ | are identical or different and represent hydrogen, fluorine, chlorine, bromine or trifluoromethyl, and |
| $R^1$ | represents $C_1$-$C_4$-alkyl or $C_1$-$C_5$-alkylcarbonyloxy-$C_1$-$C_5$-alkyl. |

4. Process for the preparation of cycloalkyl- or cycloalkenylcarboxanilide carbamates of the formula

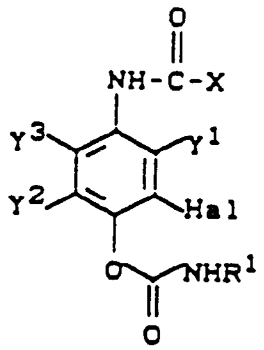

(I)

in which

X, Hal, $Y^1$, $Y^2$, $Y^3$ and $R^1$ have the abovementioned meanings,

characterised in that aminophenols of the formula

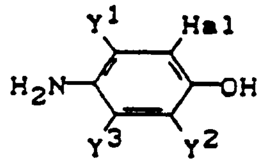

(II)

in which

Hal, $Y^1$, $Y^2$ and $Y^3$ have the abovementioned meanings, are reacted, in a first reaction step, with carboxylic acid derivatives of the formula

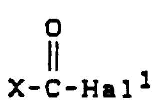

(III)

in which

X has the abovementioned meaning and

$Hal^1$ represents halogen or a leaving group customary in acylation reactions,

if appropriate in the presence of an acid acceptor and if appropriate in the presence of a solvent or diluent, and, in a second reaction step, the resulting intermediates of the formula

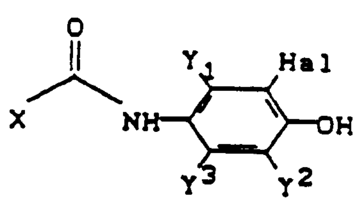

(IV)

in which

X, $Y^1$, $Y^2$, $Y^3$ and Hal have the abovementioned meaning,

are then reacted with isocyanates of the formula (V)

$R^1$-NCO (V)

in which

$R^1$ has the abovementioned meaning,

if appropriate in the presence of a base and if appropriate in the presence of diluents or solvents.

5. Pesticides, characterised in that they contain at least one cycloalkyl- or cycloalkenyl-carboxanilide carbamate of the formula (I) according to Claim 1.

6. Use of cycloalkyl- or cycloalkenyl-carboxanilide carbamates of the formula (I) according to Claim 1, for combating pests.

7. Method of combating pests, characterised in that cycloalkyl- or cycloalkenyl-carboxanilide carbamates of the formula (I) according to Claim 1 are allowed to act on pests and/or their habitat.

8. Process for the preparation of pesticides, characterised in that cycloalkyl- or cycloalkenylcarboxanilide carbamates of the formula (I) according to Claim 1 are mixed with extenders and/or surface-active agents.

## Revendications

1. Anilidocarbamates d'acides cycloalkyl- ou cycloalcénylcarboxyliques répondant à la formule

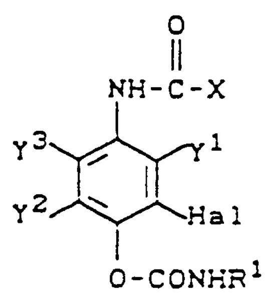

(I)

dans laquelle

| | |
|---|---|
| X | représente un groupe cyclopropyle, un groupe cyclobutyle, un groupe cyclopentyle, un groupe cyclohexyle ou un groupe cycloheptyle substitués de 1 à 6 fois de manière identique ou différente par un groupe alkyle à chaîne droite ou ramifiée contenant de 1 à 4 atomes de carbone, ou représente un groupe cycloalcényle contenant de 5 à 7 termes cycliques, le radical cycloalcényle pouvant être substitué de 1 à 6 fois de manière identique ou différente par un groupe alkyle à chaîne droite ou ramifiée contenant de 1 à 4 atomes de carbone, |
| Hal | représente un atome de fluor, un atome de chlore ou un atome de brome, |
| $Y^1$, $Y^2$ et $Y^3$ | sont identiques ou différents et représentent un atome d'hydrogène, un atome de fluor, un atome de chlore, un atome de brome, un groupe alkyle à chaîne droite ou ramifiée contenant de 1 à 4 atomes de carbone, un groupe alcoxy ou un groupe alkylthio à chaîne droite ou ramifiée contenant chacun de 1 à 4 atomes de carbone; un groupe halogénalkyle, un groupe halogénalcoxy ou un groupe halogénalkylthio contenant chacun de 1 à 4 atomes de carbone dans la fraction alkyle à chaîne droite ou ramifiée et contenant de 1 à 5 atomes d'halogène identiques ou différents, et |
| $R^1$ | représente un groupe alkyle en $C_1$-$C_8$ ou encore un groupe alkyl(en $C_1$-$C_8$)carbonyloxyalkyle en $C_1$-$C_6$. |

2. Composés de formule (I) selon la revendication 1,

dans lesquels :

| | |
|---|---|
| X | représente un groupe cyclopropyle, un groupe cyclobutyle, un groupe cyclopentyle, un groupe cyclohexyle ou un groupe cycloheptyle substitués une ou deux fois de manière identique ou différente par un groupe alkyle à chaîne droite ou ramifiée conte- |

nant de 1 à 4 atomes de carbone; un groupe cyclopenténylе, un groupe cyclohexényle, un groupe cycloheptényle éventuellement substitués une ou deux fois de manière identique ou différente par un groupe alkyle à chaîne droite ou ramifiée contenant de 1 à 4 atomes de carbone,

$Y^1$, $Y^2$ et $Y^3$ sont identiques ou différents et représentent un atome d'hydrogène, un atome de fluor, un atome de chlore, un atome de brome, un groupe méthyle ou un groupe trifluorométhyle, et

Hal représente un atome de fluor, un atome de chlore ou un atome de brome, et

$R^1$ représente un groupe alkyle en $C_1$-$C_6$ ou un groupe alkyl(en $C_1$-$C_6$)carbonyloxyalkyle en $C_1$-$C_6$.

3. Composés de formule (I) selon la revendication 1, dans lesquels

X représente un groupe cyclopentyle, un groupe cyclohexyle, un groupe cycloheptyle, un groupe cyclopentenyle, un groupe cyclohexanyle ou un groupe cycloheptenyle toutes les radicales sont substitués dans la position 1 ou 1,3 par méthyle ou éthyle, et toutes les radicals sont éventuellement substitués additionelles par un groupe alkyle contenant 1 à 3 atomes de carbone,

Hal représente un atome de fluor, un atome de chlore ou un atome de brome,

$Y^1$, $Y^2$, et $Y^3$ sont identiques ou différents et représentent un atome d'hydrogène, un atome de fluor, un atome de chlore, un atome de brome ou un groupe de trifluorométhyle, et

$R^1$ représente un groupe alkyle en $C_1$-$C_4$ ou un groupe alkyl (en $C_1$-$C_5$) carbonyloxyalkyle en $C_1$-$C_5$.

4. Procédé de préparation d'anilidocarbamates d'acides cycloalkyl- ou cycloalcénylcarboxyliques répondant à la formula

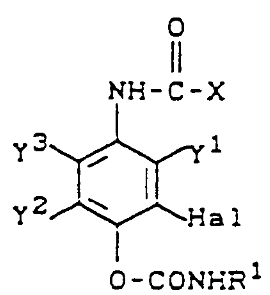

(I)

dans laquelle

X, Hal, $Y^1$, $Y^2$, $Y^3$ et $R^1$ ont les significations indiquées ci-dessus,

caractérisé en ce qu'on fait réagir des aminophénols de formule

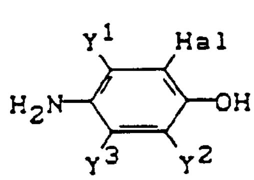

(II)

dans laquelle

Hal, $Y^1$, $Y^2$ et $Y^3$ ont les significations indiquées ci-dessus, dans une première étape réactionnelle avec des dérivés d'acides carboxyliques répondant à la formule

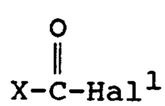

(III)

dans laquelle

X a la signification indiquée ci-dessus et

$Hal^1$ représente un atome d'halogène ou un groupe habituel qui se sépare lors de réactions d'acylation éventuellement en présence d'un accepteur d'acides et éventuellement en présence d'un solvant ou d'un diluant, et en ce qu'on fait ensuite réagir, dans une seconde étape réactionnelle, les produits intermédiaires ainsi obtenus répondant à la formule

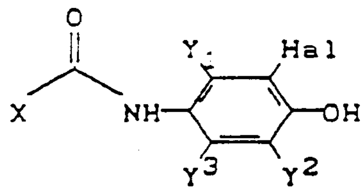

(IV)

dans laquelle

X, $Y^1$, $Y^2$, $Y^3$ et Hal ont la signification indiquée,

avec des isocyanates de formule

$R^1$-NCO (V)

dans laquelle

$R^1$ a la signification indiquée ci-dessus, éventuellement en présence d'une base et éventuellement en présence de diluants ou de solvants.

**5.** Agent de lutte contre les parasites, qui se caractérise par une teneur en au moins un anilidocarbamate d'acide cycloalkyl- ou cycloalcényl-carboxylique de formule (I) selon la revendication 1.

**6.** Utilisation d'anilidocarbamates d'acides cycloalkyl- ou cycloalcényl-carboxyliques de formule (I) selon la revendication 1, pour lutter contre les parasites.

**7.** Procédé pour lutter contre les parasites, caractérisé en ce qu'on laisse agir des anilidocarbamates d'acides cycloalkyl- ou cycloalcényl-carboxyliques de formule (I) selon la revendication 1, sur des parasites et/ou sur leur biotope.

**8.** Procédé pour la préparation d'agents de lutte contre les parasites, caractérisé en ce qu'on mélange des anilidocarbamates d'acides cycloalkyl- ou cycloalcénylcarboxyliques de formule (I) selon la revendication 1, avec des diluants et/ou des agents tensioactifs.